# EUROPÄISCHE PATENTSCHRIFT

(11) **EP 1 028 696 B1**
(45) Veröffentlichungstag und Bekanntmachung des Hinweises auf die Patenterteilung: **28.08.2002**
(21) Anmeldenummer: 98959817.2
(22) Anmeldetag: 30.10.1998
(51) Int. Cl.: A61K 7/00

(54) **VERWENDUNG VON MISCHUNGEN ZUR HERSTELLUNG VON ABSCHMINKMITTELN**
USE OF MIXTURES FOR PRODUCING MAKE-UP REMOVERS
UTILISATION DE MELANGES POUR LA FABRICATION DE DEMAQUILLANTS

(30) Priorität: 10.11.1997 DE 19749550
(43) Veröffentlichungstag der Anmeldung: 23.08.2000
(73) Patentinhaber: Cognis Deutschland GmbH & Co. KG, 40589 Düsseldorf (DE)
(72) Erfinder: WADLE, Armin, D-40699 Erkrath (DE); LE HEN-FERRENBACH, Catherine, F-77100 Meaux (FR)
(86) Internationale Anmeldenummer: EP9806897
(87) Internationale Veröffentlichungsnummer: WO99023999

(56) Entgegenhaltungen:
- EP-A- 0 422 862
- WO-A-93/00089
- FR-A- 1 500 775
- US-A- 4 806 572

## Beschreibung

### Gebjet der Erfindung

Die Erfindung betrifft die Verwendung von Mischungen, enthaltend Monoglycerid(ether)sulfate, Ölkörper und gegebenenfalls Fettalkohole zur Herstellung von Abschminkmitteln.

### Stand der Technik

Im Bereich der dekorativen Kosmetik finden die unterschiedlichsten Einsatzstoffe Verwendung. Eine typische Make-up-Zusammensetzung enthält beispielsweise Glycerinmonostearat, Cetylalkohol, Stearinsäure, Paraffinöl, Cetylstearyloctanoat, Octylpalmitat, Talkum, Titandioxid, Eisenoxide, Propylenglycol, Polysorbate, Xanthan, Magnesium-Aluminiumsilicat, Glycerin, Parfümöle, Konservierungsmittel und Wasser. In Wimperntusche oder Mascara sind Wachse, Farbstoffe, Emulgatoren und Verdickungsmittel enthalten. Lippenstifte enthalten neben Farbstoffen, Wachsen und Ölkörpem vor allem auch Glimmer Titandioxid und neuerdings auch Siliconverbindungen. Lidschatten stellen üblicherweise Mischungen von Farbstoffen mit Glimmer oder Titandioxid dar, die als weitere Bestandteile Talkum, Paraffinöl, Kaolin, Metallseifen, Wachsalkohole und Emulgatoren enthalten können. Kajalstifte enthalten beispielsweise Eisenoxide, Pflanzenöle, Wachse, Fettsäuren, Talkum und Emulgatoren. Entsprechende kosmetische Reinigungsmittel müssen somit eine Vielzahl völlig unterschiedlicher Stoffe möglichst vollständig von der Haut entfemen, also typische Wachse, Öle und Siliconverbindungen lösen und gleichzeitig auch Pigmente wie Glimmer oder Titandioxid solubilisieren. Des weiteren müssen sie so mild wie nur eben möglich sein, um bei den Verbraucherinnen keine Hautrötungen oder gar Augenschleimhautirritationen auszulösen. Üblicherweise werden derartige Zubereitungen, wie sie beispielsweise in der **EP-B1 0705592** (L'Oréal) offenbart sind, entweder in Form von Lotionen, gegebenenfalls auf Watteträgem, oder als Cremes angeboten. Hier besteht seitens der Verbraucherin zusätzlich der Wunsch, daß die Produkte, bei denen es sich stets um Emulsionen handelt, eine ausreichende Lagerstabilität besitzen und sich insbesondere nicht in der Wärme irreversibel entmischen.

In diesem Zusammenhang sei auf die Deutsche Patentanmeldung **DE-A1 19540829** (Henkel) verwiesen, aus der kosmetische Emulsionen bekannt sind, die neben Partialglycerid(ether)sulfaten Alkylglucoside und Fettalkohole enthalten. Die französische Patentanmeldung **FR-A1 1 500 775** beschreibt ein Verfahren zur Herstellung von Reinigungsprodukten für die Haut, die andere schäumende Reinigungsstoffe als Natriumlaurylsulfate sowie Lipoproteine und optional weitere Hilfsstoffe wie Sulfosuccinate, Polypeptide und aseptische Wirkstoffe enthalten, wobei Glyceridethersulfate in der Aufzählung möglicher Detergentien genannt ist. Gegenstand der Deutschen Patentanmeldung **DE-A1 19605360** (Henkel) sind Emulsionen mit besonders hoher Lagerstabilität, die Monoglycerid(ether)-sulfate, Emulgatoren und Chitosane enthalten. Keine der Schriften enthält jedoch einen Hinweis auf die Herstellung von Abschminkmitteln.

Demzufolge hat die komplexe Aufgabe der vorliegenden Erfindung darin bestanden, neue Hautreinigungsmittel in Emulsionsform zur Verfügung zu stellen, die ein hohes Reinigungsvermögen sowohl gegenüber Wachsen, Ölen, Siliconverbindungen als auch gegenüber Pigmenten aufweisen, außerordentlich haut- und schleimhautverträglich sind und gleichzeitig auch eine besonders hohe Lagerstabilität besitzen.

### Beschreibung der Erfindung

Gegenstand der Erfindung ist die Verwendung von Mischungen, enthaltend
(a) Monoglycerid(ether)sulfate und
(b) Ölkörper

zur Herstellung von Abschminkmitteln in Emulsionsform.

Überraschenderweise wurde gefunden, daß Mischungen aus Monoglycerid(ether)sulfaten und Ölkörpem ein hohes Reinigungsvermögen für Wachse, Öle und Pigmente sowie insbesondere Siliconverbindungen aufweisen, dabei gut haut- und augenschleimhautverträglich sind und sich insbesondere auch in der Wärme nicht entmischen. Die Erfindung schließt die Erkenntnis ein, daß die Lagerstabilität der binären Mischungen durch Zusatz von Fettalkoholen weiter verbessert wird; gleichzeitig wird die Konsistenz der Zubereitungen erhöht.

### Monoglycerid(ether)sulfate

Monoglycerid(ether)sulfate stellen bekannte anionische Tenside dar, die nach den einschlägigen Methoden der präparativen organischen Chemie erhalten werden können. Üblicher-weise geht man zu ihrer Herstellung von Triglyceriden aus, die gegebenenfalls nach Ethoxylierung zu den Monoglyceriden umgeestert und nachfolgend sulfatiert und neutralisiert werden. Gleichfalls ist es möglich, die Partialglyceride mit geeigneten Sulfatierungsmitteln, vorzugsweise gasförmiges Schwefel-trioxid oder Chlorsulfonsäure umzusetzen [vgl. **EP-B1 0561825, EP-B1 0561999** (Henkel)]. Die neutralisierten Stoffe können - falls gewünscht - einer Ultrafiltration unterworfen werden, um den Elektrolyt-gehalt auf ein gewünschtes Maß zu vermindern **[DE-A1 4204700** (Henkel)]. Übersichten zur Chemie der Monoglycerid(ether)sulfate sind beispielsweise von A.K.Biswas et al. in **J.Am.Oil.Chem.Soc. 37, 171 (1960)** und F.U.Ahmed **J.Am.Oil.Chem.Soc. 67, 8 (1990)** erschienen. Die im Sinne der Erfindung einzusetzenden Monoglycerid(ether)sulfate folgen der Formel **(I)**, 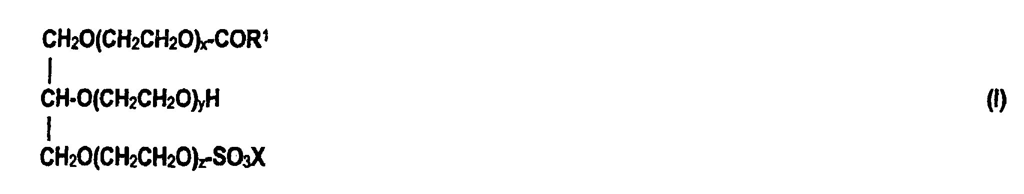 in der R¹CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Cetylstearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglycerid(ether)sulfate der Formel **(I)** eingesetzt, in der R¹CO für einen linearen Acylrest mit 12 bis 14 oder 16 bis 18 Kohlenstoffatomen steht.

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare C₆-C₂₂-Fettalkoholcarbonate, Guerbetcarbonate, Ester der Benzoesäure mit linearen und/oder verzweigten C₆-C₂₂-Alkoholen (z.B. Finsolv® TN), Dialkylether, Ringöffnungsprodukte von epoxidierten Fettsäureestem mit Polyolen, Siliconöle und/oder aliphatische bzw. naphthenische Kohlenwasserstoffe in Betracht.

### Fettalkohole

Unter Fettalkoholen sind primäre aliphatische Alkohole der Formel **(II)** zu verstehen,

**R**^{**2**}**OH (II)**

in der R² für einen aliphatischen, linearen oder verzweigten Kohlenwasserstoffrest mit 6 bis 22 Kohlenstoffatomen und 0 und/oder 1, 2 oder 3 Doppelbindungen steht. Typische Beispiele sind Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestem auf Basis von Fetten und Ölen oder Aldehyden aus der Roelen'schen Oxosynthese sowie als Monomerfraktion bei der Dimerisierung von . ungesättigten Fettalkoholen anfallen. Bevorzugt sind technische Fettalkohole mit 12 bis 18 Kohlenstoffatomen wie beispielsweise Kokos-, Palm-, Palmkem- oder Talgfettalkohol. In einer besonders bevorzugten Ausführungsform der Erfindung werden Fettalkohole eingesetzt, die den gleichen Fettrest wie die Monoglycerid(ether)sulfate aufweisen, also beispielsweise Cetylstearylalkohol in Kombination mit Cetylstearinsäuremonoglyceridsulfat.

### Abschminkmittel

In einer bevorzugten Ausführungsform betrifft ein weiterer Gegenstand der Erfindung die Verwendung von Mischungen, enthaltend
(a) 1 bis 10, vorzugsweise 2 bis 5 Gew.-% Monoglycerid(ether)sulfate,
(b) 10 bis 80, vorzugsweise 20 bis 50 Gew.-% Ölkörper, sowie
(c) 0 bis 20, vorzugsweise 5 bis 20 Gew.-% Fettalkohole, zur Herstellung von Abschminkmitteln für die Augen und empfindliche Hautpartien, mit der Maßgabe, daß sich die Mengenangaben mit Wasser und weiteren üblichen Hilfs- und Zusatzstoffen zu 100 Gew.-% ergänzen. Die unter Verwendung der Mischungen hergestellten Mittel weisen in der Regel einen Wasseranteil von 10 bis 90, vorzugsweise 20 bis 70 Gew.-% auf und können dabei sowohl als dünnflüssige, sprühbare Emulsionen mit Tröpfchengrößen der Ölphase von < 2 µm oder als Cremes vorliegen. In einer speziellen Ausführungsform werden zur Herstellung von Augenreinigungsmitteln oder Abschminktüchem geeignete Träger, wie beispielsweise Watte oder Viscose mit den Zubereitungen getränkt und so in den Handel gebracht.

### Gewerbliche Anwendbarkeit

Die unter Verwendung der Mischungen erhältlichen Zubereitungen können des weiteren als Hilfs- und Zusatzstoffe milde Tenside, Co-Emulgatoren, Überfettungsmittel, Stabilisatoren, Konsistenzgeber, Verdickungsmittel, Siliconverbindungen, biogene Wirkstoffe, Konservierungsmittel, Hydrotrope, Solubilisatoren, Parfümöle, Farbstoffe und dergleichen enthalten.

Typische Beispiele für geeignete milde, d.h. besonders hautverträgliche Tenside sind Fettalkoholpolyglycolethersulfate, Mono- und/oder Dialkylsulfosuccinate, Fettsäureisethionate, Fettsäuresarcosinate, Fettsäuretauride, Fettsäureglutamate, Ethercarbonsäuren, Alkyloligoglucoside, Fettsäureglucamide, Alkylamidobetaine und/oder Proteinfettsäurekondensate, letztere vorzugsweise auf Basis von Weizenproteinen.

Als **Co-Emulgatoren** kommen beispielsweise nichtionogene Tenside aus mindestens einer der folgenden Gruppen in Frage:
(1) Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) C_{12/18}-Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(4) Alkylmono- und -oligoglycoside mit 8 bis 22 Kohlenstoffatomen im Alkylrest und deren ethoxylierte Analoga;
(5) Anlagerungsprodukte von 15 bis 60 Mol Ethylenoxid an Ricinusöl und/oder gehärtetes Ricinusöl;
(6) Polyol- und insbesondere Polyglycerinester wie z.B. Polyglycerinpolyricinoleat oder Polyglycerinpoly-12-hydroxystearat. Ebenfalls geeignet sind Gemische von Verbindungen aus mehreren dieser Substanzklassen;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
(9) Trialkylphosphate sowie Mono-, Di- und/oder Tri-PEG-alkylphosphate;
(10) Wollwachsalkohole;
(11) Polysiloxan-Polyalkyl-Polyether-Copolymere bzw. entsprechende Derivate;
(12) Mischester aus Pentaerythrit, Fettsäuren, Citronensäure und Fettalkohol gemäß **DE-PS 1165574** undloder Mischester von Fettsäuren mit 6 bis 22 Kohlenstoffatomen, Methylglucose und Polyolen, vorzugsweise Glycerin sowie
(13) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 20 24 051** als Rückfettungsmittel für kosmetische Zubereitungen bekannt.

C_{8/18}-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquatemierte Difettsäuretriethanolaminester-Salze, besonders bevorzugt sind.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als Konsistenzgeber kommen in erster Linie Fettalkohole mit 12 bis 22 und vorzugsweise 16 bis 18 Kohlenstoffatomen und daneben Partialglyceride in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdlckungsmittel** sind beispielsweise Polysaccharide, insbesondere Xanthan-Gum, Guar-Guar, Agar-Agar, Alginate und Tylosen, Carboxymethylcellulose und Hydroxyethylcellulose, femer höhermolekulare Polyethylenglycolmono- und -di-ester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Typische Beispiele für **Fette** sind Glyceride, als **Wachse** kommen u.a. Bienenwachs, Camaubawachs, Candelillawachs, Montanwachs, Paraffinwachs oder Mikrowachse gegebenenfalls in Kombination mit hydrophilen Wachsen, z.B. Cetylstearylalkohol oder Partialglyceriden in Frage. Als **Stabilisatoren** können Metallsalze von Fettsäuren wie z.B. Magnesium-, Aluminium- und/oder Zinkstearat eingesetzt werden. Unter **biogenen Wirkstoffen** sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen. Als Quellmittel für wäßrige Phasen können Montmorillonite, Clay Mineralstoffe, Pemulen sowie alkylmodifizierte Carbopoltypen (Goodrich) dienen.

Zur Verbesserung des Fließverhaltens können femer **Hydrotrope** wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Typische Beispiele sind
- Glycerin;
- Alkylenglycole wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche, mit 1 bis 8 Kohlenstoffen im Alkylrest wie beispielsweise Methyl- und Butylglucosid;
- Zuckeralkohole mit 5 bis 12 Kohlenstoffatomen wie beispielsweise Sorbit oder Mannit,
- Zucker mit 5 bis 12 Kohlenstoffatomen wie beispielsweise Glucose oder Saccharose;
- Aminozucker wie beispielsweise Glucamin.

Als **Konservierungsmittel** eignen sich beispielsweise Phenoxyethanol, Formaldehydlösung, Parabene, Pentandiol oder Sorbinsäure. Als **Parfümöle** seien genannt die Extrakte von Blüten (Lavendel, Rosen, Jasmin, Neroli), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Sandel-, Guajak-, Zedem-, Rosenholz), Kräutem und Gräsem (Estragon, Lemon-gras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Moschus, Zibet und Castoreum. Als synthetische bzw. halbsynthetische Parfümöle kommen Ambroxan, Eugenol, Isoeugenol, Citronellal, Hydroxycitronellal, Geraniol, Citronellol, Geranylacetat, Citral, lonon und Methylionon in Betracht.

Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt- oder Heißprozesse erfolgen; vorzugsweise arbeitet man nach der Phaseninversionstemperatur-Methode.

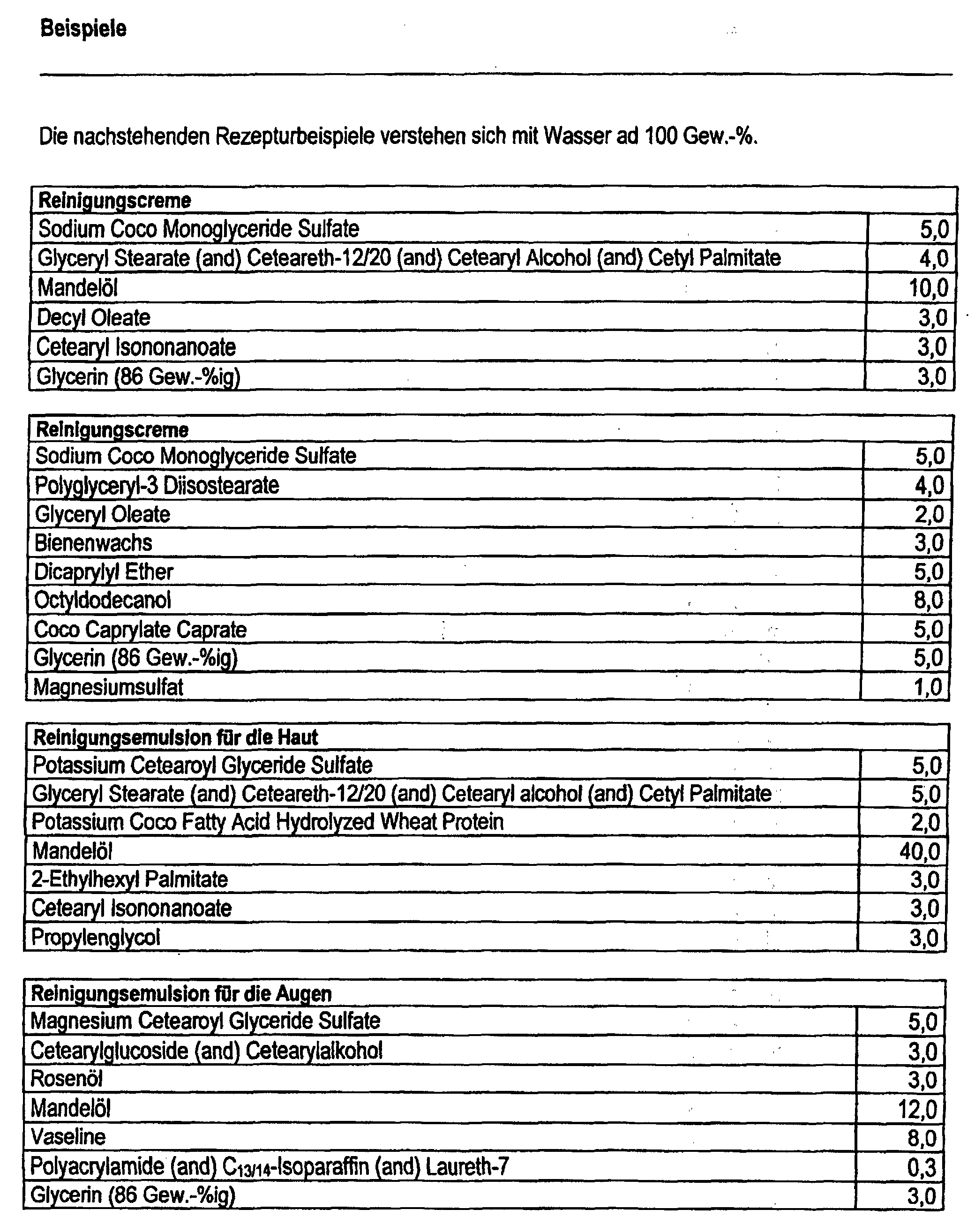

## Patentansprüche

1. Verwendung von Mischungen, enthaltend
(a) Monoglycerid(ether)sulfate und
(b) Ölkörper
zur Herstellung von Abschminkmitteln in Emulsionsform.

2. Verwendung nach Anspruch 1, **dadurch gekennzeichnet, daß** man Monoglycerid(ether)sulfate der Formel **(I)** einsetzt, 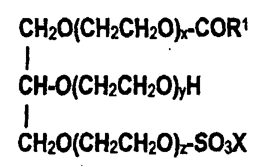 in der R¹CO für einen linearen oder verzweigten Acylrest mit 6 bis 22 Kohlenstoffatomen, x, y und z in Summe für 0 oder für Zahlen von 1 bis 30 und X für ein Alkali- oder Erdalkalimetall steht.

3. Verwendung nach den Ansprüchen 1 und 2, **dadurch gekennzeichnet, daß** man Monoglycerid (ether)sulfate der Formel **(I)** einsetzt, in der R¹CO für einen Alkylrest mit 12 bis 14 Kohlenstoffatomen, x, y und z für 0 und X für Natrium, Kalium oder Magnesium steht.

4. Verwendung nach den Ansprüchen 1 und 2, **dadurch gekennzeichnet, daß** man Monoglycerid(ether)sulfate der Formel **(I)** einsetzt, in der R¹CO für einen Alkylrest mit 16 bis 18 Kohlenstoffatomen, x, y und z für 0 und X für Natrium, Kalium oder Magnesium steht.

5. Verwendung nach den Ansprüchen 1 bis 4, **dadurch gekennzeichnet, daß** man Ölkörper einsetzt, die ausgewählt sind aus der Gruppe, die gebildet wird von Guerbetalkoholen auf Basis von Fettalkoholen mit 6 bis 18, vorzugsweise 8 bis 10 Kohlenstoffatomen, Estem von linearen C₆-C₂₀-Fettsäuren mit linearen C₆-C₂₀-Fettalkoholen, Estern von verzweigten C₆-C₁₃-Carbonsäuren mit linearen C₆-C₂₀-Fettalkoholen, Estem von linearen C₆-C₁₈-Fettsäuren mit verzweigten Alkoholen, Estem von linearen und/oder verzweigten Fettsäuren mit mehrwertigen Alkoholen und/oder Guerbetalkoholen, Triglyceriden auf Basis C₆-C₁₀-Fettsäuren, Estem von C₆-C₂₂-Fettalkoholen und/oder Guerbetalkoholen mit aromatischen Carbonsäuren pflanzlichen Ölen, verzweigten primären Alkoholen, substituierten Cyclohexanen, Guerbetcarbonaten, Dialkylethen, Ringöffnungsprodukten von epoxidierten Fettsäureestem mit Polyolen, Siliconölen und/oder aliphatischen bzw. naphthenischen Kohlenwasserstoffen.

6. Verwendung nach den Ansprüchen 1 bis 5, **dadurch gekennzeichnet, daß** die Mischungen weiterhin Fettalkohole der Formel **(II)** enthalten,
**R**^{**2**}**OH (II)**
in der R² für einen aliphatischen, linearen oder verzweigten Kohlenwasserstoffrest mit 6 bis 22 Kohlenstoffatomen und 0 und/oder 1, 2 oder 3 Doppelbindungen steht.

7. Verwendung nach Anspruch 6, **dadurch gekennzeichnet, daß** man Fettalkohole einsetzt, die den gleichen Fettrest wie die Monoglycerid(ether)sulfate aufweisen.

8. Verwendung nach den Ansprüchen 1 bis 7, **dadurch gekennzeichnet, daß** man
(a) 1 bis 10 Gew.-% Monoglycerid(ether)sulfate,
(b) 10 bis 80 Gew.-% Ölkörper
(c) 0 bis 20 Gew.-% Fettalkohole
einsetzt, mit der Maßgabe, daß sich die Mengenangaben mit Wasser und weiteren üblichen Hilfsund Zusatzstoffen zu 100 Gew.-% ergänzen.

## Claims

1. The use of mixtures containing
(a) monoglyceride (ether) sulfates and
(b) oil components
for the production of make-up removers in emulsion form.

2. The use claimed in claim 1, **characterized in that** monoglyceride (ether) sulfates corresponding to formula **(I)**: 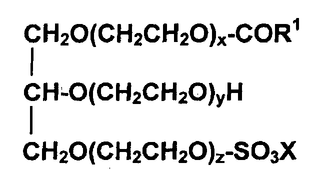 in which R¹CO is a linear or branched acyl group containing 6 to 22 carbon atoms, x, y and z add up to 0 or to a number of 1 to 30 and X is an alkali metal or alkaline earth metal, are used.

3. The use claimed in claims 1 and 2, **characterized in that** monoglyceride (ether) sulfates corresponding to formula **(I)**, in which R¹CO is a C₁₂₋₁₄ alkyl group, x, y and z = 0 and X stands for sodium, potassium or magnesium, are used.

4. The use claimed in claims 1 and 2, **characterized in that** monoglyceride (ether) sulfates corresponding to formula **(I)**, in which R¹CO is a C₁₆₋₁₈ alkyl group, x, y and z = 0 and X stands for sodium, potassium or magnesium, are used.

5. The use claimed in claims 1 to 4, **characterized in that** oil components selected from the group consisting of Guerbet alcohols based on fatty alcohols containing 6 to 18 and preferably 8 to 10 carbon atoms, esters of linear C₆₋₂₀ fatty acids with linear C₆₋₂₀ fatty alcohols, esters of branched C₆₋₁₃ carboxylic acids with linear C₆₋₂₀ fatty alcohols, esters of linear C₆₋₁₈ fatty acids with branched alcohols, esters of linear and/or branched fatty acids with polyhydric alcohols and/or Guerbet alcohols, triglycerides based on C₆₋₁₀ fatty acids, esters of C₆₋₂₂ fatty alcohols and/or Guerbet alcohols with aromatic carboxylic acids, vegetable oils, branched primary alcohols, substituted cyclohexanes, Guerbet carbonates, dialkyl ethers, ring opening products of epoxidized fatty acid esters with polyols, silicone oils and/or aliphatic or naphthenic hydrocarbons are used.

6. The use claimed in claims 1 to 5, **characterized in that** the mixtures additionally contain fatty alcohols corresponding to formula **(II)**:
**R**^{**2**}**OH (II)**
in which R² is an aliphatic, linear or branched hydrocarbon radical containing 6 to 22 carbon atoms and 0 and/or 1, 2 or 3 double bonds.

7. The use claimed in claim 6, **characterized in that** fatty alcohols with the same fatty residue as the monoglyceride (ether) sulfates are used.

8. The use claimed in claims 1 to 7, **characterized in that**
(a) 1 to 10% by weight of monoglyceride (ether) sulfates,
(b) 10 to 80% by weight of oil components,
(c) 0 to 20% by weight of fatty alcohols
with the proviso that the quantities shown add up to 100% by weight with water and other typical auxiliaries and additives,
are used.

## Revendications

1. Utilisation de mélanges contenant
(a) des (éther)sulfates de monoglycérides et
(b) des corps huileux
pour la préparation d'agents démaquillants sous forme d'émulsion.

2. Utilisation selon la revendication 1,
**caractérisée en ce qu'**
on emploie des (éther)sulfates de monoglycérides de formule (I), 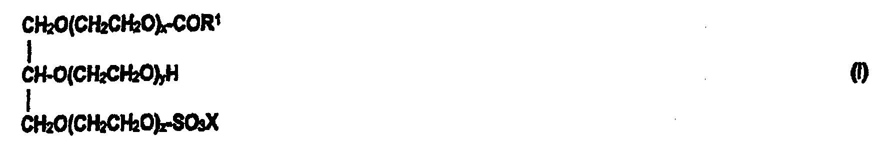 dans laquelle R¹CO représente un radical acyle linéaire ou ramifié comportant de 6 à 22 atomes de carbone, x, y et z valent au total O ou des nombres allant de 1 à 30, et X représente un métal alcalin ou alcalino-terreux.

3. Utilisation selon les revendications 1 et 2,
**caractérisée en ce qu'**
on utilise des (éther)sulfates de monoglycéride de formule (I) dans laquelle R¹CO représente un radical alkyle comportant de 12 à 14 atomes de carbone, x, y et z valent O et X représente le sodium, le potassium ou le magnésium.

4. Utilisation selon les revendication 1 et 2,
**caractérisée en ce qu'**
on utilise des éther(sulfates) de monoglycérides de formule (I) dans laquelle R¹CO représente un radical alkyle comportant de 16 à 18 atomes de carbone, x, y et z valent O et X représente le sodium, le potassium ou le magnésium.

5. Utilisation selon les revendications 1 à 4,
**caractérisée en ce qu'**
on utilise des corps huileux qui sont choisis dans le groupe formé par les alcools de Guerbet à base d'alcools gras comportant de 6 à 18, de préférence de 8 à 10 atomes de carbone ; les esters d'acides gras en C₆ à C₂₀ linéaires avec des alcools gras en C₆ à C₂₀ linéaires, les esters d'acides carboxyliques en C₆ à C₁₃ ramifiés avec des alcools gras en C₆ à C₂₀ linéaires, les esters d'acides gras en C₆ à C₁₈ linéaires avec des alcools ramifiés, les esters d'acides gras linéaires et/ou ramifiés avec des alcools polyvalents et/ou les alcools de Guerbet, les triglycérides à base d'acides gras en C₆ à C₁₀, les esters d'alcools gras en C₆ à C₂₂ et/ou des alcools de Guerbet avec des acides carboxyliques aromatiques d'huiles végétales, des alcools primaires ramifiés, des cyclohexanes substitués, des carbonates de Guerbet, des éthers de dialkyle, des produits d'ouverture de cycle d'esters d'acides gras époxydés avec des polyols, des huiles de silicones et/ou des hydrocarbures aliphatiques ou selon les cas naphténiques.

6. Utilisation selon les revendications 1 à 5,
**caractérisée en ce que**
les mélanges contiennent en outre des alcools gras de formule (II)
R²OH (II)
dans laquelle R² représente un radical hydrocarboné, aliphatique, linéaire ou ramifié comportant de 6 à 22 atomes de carbone et 0 et/ou 1, 2 ou 3 doubles liaisons.

7. Utilisation selon la revendication 6,
**caractérisée en ce qu'**
on utilise des alcools gras qui présentent le même radical gras que les (éther)sulfates de monoglycéride.

8. Utilisation selon les revendications 1 à 7,
**caractérisée en ce qu'**
on utilise
(a) de 1 à 10 % en poids d'(éther)sulfate de monoglycéride,
(b) de 10 à 80 % en poids de corps huileux,
(c) de 0 à 20 % en poids d'alcools gras
sous réserve que les indications quantitatives se complètent à 100 % avec de l'eau et d'autres adjuvants et additifs habituels.
